# FASCICULE DE BREVET EUROPEEN

(11) **EP 1 469 845 B1**
(45) Date de publication et mention de la délivrance du brevet: **15.08.2007**
(21) Numéro de dépôt: 03712286.8
(22) Date de dépôt: 31.01.2003
(51) Int. Cl.: A61K 31/245, A61K 31/17, C07C 275/54, C07C 233/87, A61P 33/02

(54) **DERIVES DE L'ACIDE HALOACETAMIDOBENZOIQUE ET LEUR UTILISATION POUR LE TRAITEMENT DE MALADIES PARASITAIRES**
HALOACETAMIDOBENZOESÄURE DERIVATE UND DEREN VERWENDUNG ZUR BEHANDLUNG VON PARASITÄREN ERKRANKUNGEN
DERIVATIVES OF HALOACETAMIDOBENZOIC ACID AND USE THEREOF FOR THE TREATMENT OF PARASITIC DISEASES

(30) Priorité: 31.01.2002 FR 0201151
(43) Date de publication de la demande: 27.10.2004
(73) Titulaire: Kalil-Fihlo, Jorge Elias, Sao Paulo SP (BR); Lepape, Patrice, 44120 Vertou (FR); Fellous, Esther S. A., 75005 Paris (FR)
(72) Inventeur: LEPAPE, Patrice, F-44120 Vertou (FR); FELLOUS, Esther, Suzy, Arlette, F-75005 Paris (FR); BEKESI, Julius, George, Gastonia, NC 28055 (US)
(74) Mandataire: Laget, Jean-Loup
(86) Numéro de dépôt international: PCT/FR2003/000289
(87) Numéro de publication internationale: WO 2003/063858

(56) Documents cités:
- EP-A- 1 008 346
- WO-A-01/16357
- WO-A-98/39323
- DE-C- 106 502
- US-A1- 2002 065 229
- US-B1- 6 294 695
- JIANG J-D ET AL: "SYNTHESIS, CANCERICIDAL AND ANTIMICROTUBULE ACTIVITIES OF 3-(HALOACETAMIDO)-BENZOYLUREAS" ANTI-CANCER DRUG DESIGN, BASINGSTOKE, GB, vol. 7, no. 13, octobre 1998 (1998-10), pages 735-747, XP001070272 ISSN: 0266-9536
- HAVENS C G ET AL: "CELLULAR EFFECTS OF LEISHMANIAL TUBULIN INHIBITORS ON L. DONOVANI" MOLECULAR AND BIOCHEMICAL PARASITOLOGY, ELSEVIER SCIENCE PUBLISHERS, AMSTERDAM, NL, vol. 110, no. 2, octobre 2000 (2000-10), pages 223-236, XP001100700 ISSN: 0166-6851
- DATABASE WPI Section Ch, Week 197807 Derwent Publications Ltd., London, GB; Class E14, AN 1978-12819a XP002217586 & JP 50 140413 A (KISSEI YAKUHIN KOGYO KK) , 11 novembre 1975 (1975-11-11)
- DAVIS ET AL.: "Novel suicide ligands of tubulin arrest cancer cells in S-phase" NEOPLASIA, vol. 1, no. 6, décembre 1999 (1999-12), pages 498-507, XP008009460 new york, usa
- GURSU ET AL.: "The reaction of N-chloroacetylbenzamide with some aromatic amines and synthesis of some quaternary pyridinium compounds" ISTANBUL UNIV. ECZACILIK FAK. MECM., vol. 17, 1981, pages 119-134, XP001115061
- NOUR EL-DIN H M ET AL: "SYNTHESIS OF CERTAIN M-AMINO BENZOIC AND METHYL ESTER DERIVATIVES OF EXPECTED LOCAL ANAESTHETIC ACTIVITY" BULLETIN OF THE FACULTY OF PHARMACY (CAIRO UNIVERSITY), XX, XX, vol. 10, no. 1, 1973, pages 307-312, XP008009493
- DATABASE CAPLUS [en ligne] CHEMICAL ABSTRACTS SERVICE, COLUMBUS, OHIO, US; EPSTEIN ET AL.: "Simple antimetabolites of Vitamin B12" retrieved from STN Database accession no. 1963:417023 XP002245336 & J. PROTOZOOLOGY, vol. 10, 1963, pages 63-73,
- DATABASE CAPLUS [en ligne] CHEMICAL ABSTRACTS SERVICE, COLUMBUS, OHIO, US; YOUSSEF ET AL.: "Unsymmetrical N,N-disubstituted anilines: synthesis of .alpha.-(N-methylanilino)acetamides" retrieved from STN Database accession no. 1976:523521 XP002245337 & INDIAN J. CHEM. SECTION B : ORGANIC CHEMSITRY INCLUDING MEDICAL CHEMISTRY, vol. 14b, no. 4, 1976, pages 279-281,

## Description

La présente invention concerne l'utilisation de composés, en particulier de dérivés de l'acide haloacétamidobenzoïque pour le traitement de maladies parasitaires, en particulier de protozooses et plus particulièrement pour le traitement de la leishmaniose, du paludisme et des tripanosomiases.

Le paludisme est une erythropathie due à *Plasmodium* transmis par un moustique. Plus de 2 milliards de sujets sont exposés au risque de transmission. Selon l'OMS, il y aurait 300 millions de nouveau cas par an et on estime à quelques millions le nombre de décès par an dans la seule Afrique noire. Toute les 9 secondes, un sujet meurt de paludisme. L'une des espèces, *Plasmodium falciparum,* est responsable du neuropaludisme, encéphalopathie mortelle en l'absence de traitement. L'apparition de la résistance à la chloroquine dans de larges régions géographiques a bouleversé la prophylaxie et le traitement de cette infection.

La maladie de Chagas est une anthropozoonose due à *Trypanosoma cruzi* transmise par des punaises hématophages, les réduves. Elle sévit en Amérique latine où elle atteint 20 millions d'individus. Cette maladie se manifeste par des méningo-encéphalites parfois mortelles ainsi que des cardiopathies et encéphalopathies chroniques. Le nombre de décès est évalué à 50000 par an. Le traitement antiparasitaire repose sur le nifurtimox (LAMPIT^{®}) d'activité très variable sur la phase aiguë de la maladie et inactif sur la phase chronique.

Conventionnellement, les leishmanioses sont décrites comme des protozooses du système réticulo-endothélial des mammifères, dont l'homme, dues au genre Leishmania. La maladie sévit dans la quasi-totalité du globe (88 pays) constituant un problème majeur de santé publique. Dans les zones d'endémie, la prévalence est estimée à plus de 12 millions de cas et plus de 400 000 nouveaux cas apparaissent chaque année.

A l'heure actuelle, 18 espèces sont reconnues pathogènes pour l'homme. *Leishmania donovani* et *L*. *infantum* sont responsables de la leishmaniose viscérale, forme clinique potentiellement fatale, dans le bassin méditerranéen, le Maghreb, l'Afrique de l'Est, l'Inde et l'Amérique du Sud. En 1993, la dernière épidémie au Soudan s'est soldée par 40 000 décès. Les agents de la leishmaniose cutanée sont *L*. *major, L. tropica* et *L*. *aetiopica* dans l'ancien monde et *L*. *mexicana, L. Panamensis* et *L*. *braziliensis* dans le nouveau monde. Ces protozoaires généralement transmis par piqûre d'insectes rentrent dans les cellules telles que les macrophages et les monocytes et provoquent un éclatement des cellules. La forme cutanée provoque des ulcères tandis que la forme viscérale provoque des fièvres, un amaigrissement, une splénomégalie.

Le traitement des leishmanioses repose essentiellement sur l'utilisation des dérivés de l'antimoine qui nécessitent l'emploi de fortes doses, une utilisation parentérale même dans les formes exclusivement cutanées et un traitement au long cours. Ces dérivés se caractérisent également par une toxicité rénale et cardiaque. Par ailleurs, la susceptibilité des souches de *Leishmania* aux antimoniés est très variable et les cas de résistance sont de plus en plus nombreux.

L'ensemble de ces considérations montre la nécessité et l'urgence de développer des molécules qui offrent de nouvelles alternatives thérapeutiques utilisant un mécanisme d'action original.

La tubuline, protéine majeure des microtubules, représente une cible cellulaire pour de nombreuses drogues dont l'efficacité clinique est établie aussi bien dans la lutte contre le cancer que dans celle induite par de nombreux agents infectieux tels que les parasites. Les dérivés benzimidazoles, connus pour leur activité antimicrobienne sont actifs contre d'autres protozoaires comme *Trichomonas vaginalis* et *Giardia duodenalis.*

Le taxol, une autre drogue antimicrotubulaire connue aujourd'hui pour son activité anticancéreuse, bloque la réplication de *Trypanosoma cruzi* et induit un arrêt en phase G2M du cycle cellulaire de *Leishmania donovani* au stade promastigote. Bien qu'efficace contre les *Leishmania,* le taxol n'a pas retenu l'attention des thérapeutes car, si ce type de drogue est capable d'altérer les microtubules du parasite, il est aussi capable d'altérer à des degrés divers les microtubules de la cellule hôte.

Par ailleurs, on connaît, à travers US-A-6.294.695, des composés dérivés en méta et para de l'acide haloacétamidobenzoïque pour leur application en tant qu'inhibiteur de croissance de cellules tumorales. Rien ne suggère dans ce document l'utilisation de tels composés pour une application au traitement de maladies parasitaires. Par ailleurs, le tableau 1 colonne 18 de ce document montre des composés inactifs ou faiblement actifs pour une application à la cancérologie. Or, ces composés se révèlent au contraire intéressants dans le cas d'une application au traitement de maladies parasitaires comme cela sera décrit ci-après en particulier pour une molécule appelée MF 29.

D'après une publication scientifique de Jiung J-D et Al. publiée en 1998, trois paramètres clefs sont essentiels afin d'obtenir une activité cancéreuse au sein d'un composé: la nature cytotoxique de l'halogène utilisé (I>Br>Cl>F), la position méta de l'halogène, et la présence de la fonction urée. Ainsi, les composés les plus actifs afin de traiter le cancer sont le 3-iodoacétamido benzoyl urée et la 3-bomoacétamido benzoyl urée. Tout comme pour le document US-A-6.294.695, les composés les moins actifs pour traiter le cancer (par exemple le 3-chloroacétamido benzoate d'éthyle) se sont révélés être particulièrement intéressant pour le traitement des maladies parasitaires (table I).

La publication scientifique de Davis et Al. de 1999 montre également le lien entre la taille de l'halogène et l'inhibition des microtubules (Table 1) des cellules comportant des halogènes en position méta de petites tailles tels que le chlore et le fluor n'inhibe pas les microtubules quand ils sont présents à une concentration de 100 µM tandis que les dérivés comportant le brome et l'iode inhibe 50% des microtubules des cellules cancéreuses à 30 et 2,5 µM, soit à des concentrations 3 à 40 fois moins importantes. Or, il s'est avéré que les dérivés de l'acétamido benzoate d'éthyle contenant du chlore étaient très intéressants dans le traitement de maladies parasitaires.

On connaît par ailleurs à travers le document WO-A-0116357 des composés dont l'action anti-parasitaire est due à l'inhibition d'un processus enzymatique. De tels composés présentent à la fois une structure et un mécanisme d'action différents de ceux des composés décrits dans la présente invention comme l'illustre en particulier le composé 105553 de la figure 17A dans lequel un groupement cyclique remplace le groupement (CH₂)ₙ de la présente invention.

On connaît également à travers le document EP-A-1.008.346 des composés dérivés de la benzoquinone inhibiteurs du NF-KB et du TNFα. De tels composés agissent donc comme agents anti-inflammatoires en particulier en intervenant dans la production de cytokines par les lymphocytes T. De tels composés sont donc aptes à traiter les réactions inflammatoires associées aux maladies parasitaires mais sont dépourvus de tout effet sur le parasite lui-même.

La demande internationale WO 98/39323 décrit quant à elle l'utilisation de dérivés thiophènes comme agents inhibiteurs de la polymérisation de la tubuline. De tels composés sont toutefois inaptes à distinguer la tubuline du parasite de celle de la cellule hôte.

Le document US-2002/0065229 publié postérieurement à la date de dépôt de la présente demande de brevet envisage uniquement à titre d'hypothèse l'application de dérivés halogénés d'acétamidobenzoyl-éthyl-acétate au traitement de maladies parasitaires bien qu'aucun essai sur un agent parasitaire ne soit décrit dans ce document.

Le document WO92/00734 décrit l'utilisation de l'acide para-acétamidobenzoïque ou de ses sels pharmaceutiquement acceptables pour le traitement d'infections causées par le *Pneumocystis carinii, toxoplasma gondii,* les espèces du *plasmodium.* Rien ne suggère dans ce document d'utiliser des dérivés halogénés d'acétamidobenzoyl à fonction ester ou encore urée pour le traitement de maladie parasitaire.

En conclusion, aucun des documents précités ne décrit l'utilisation de composés anti-microtubulaires peu toxiques, d'activité élevée, aptes à altérer les microtubules de parasites sans altérer les microtubules de mammifères.

Un but de la présente invention est de proposer une nouvelle utilisation de composés pour le traitement des maladies parasitaires, en particulier des leishmanioses, du paludisme et des tripanosomiases, quelle que soit leur forme clinique, ces composés étant peu toxiques comparés aux médicaments connus et présentant une aptitude à être utilisés par des voies d'administration ne nécessitant pas d'hospitalisation.

Un autre but de la présente invention est de proposer une nouvelle utilisation de composés pour le traitement des maladies parasitaires, en particulier des leishmanioses, du paludisme et des tripanosomiases, ces composés entraînant des altérations de morphologie des microtubules détectées par microscopie électronique à balayage, ces altérations s'accompagnant de modifications de l'organisation de certaines classes de microtubules et d'une spécificité d'action sur les microtubules dépendant de la composition microtubulaire de la cellule qu'elles affectent.

Un autre but de la présente invention est de proposer une nouvelle utilisation de composés pour la préparation d'un médicament pour le traitement des maladies parasitaires, en particulier des leishmanioses, du paludisme et des tripanosomiases, qui perturbe la polymérisation de la tubuline du parasite sans perturber celle de la cellule hôte.

L'invention a encore pour objet l'utilisation d'un composé de formule générale (I), de ses énantiomères, de ses diastéréoisomères et de ses sels d'addition à un acide pharmaceutiquement acceptable pour la production d'un médicament incluant au moins un agent anti-microtubulaire actif pour le traitement de protozooses du système réticulo endothélial des mammifères.

L'invention a encore pour objet l'utilisation d'un composé de formule générale (I), de ses énantiomères, de ses diastéréoisomères et de ses sels d'addition à un acide pharmaceutiquement acceptable pour la production d'un agent anti-microtubulaire actif pour le traitement des leishmanioses, en particulier de la leishmaniose humaine, du paludisme et des tripanosomiases.

Il est à noter que dans ce qui précède, parmi les acides pharmaceutiquement acceptables, on peut citer, à titre non limitatif, les acides chlorhydrique, sulfurique, tartrique, maléique, fumarique, oxalique, méthane-sulfonique, camphorique, éthane-sulfonique, etc...

Les termes alkyle et alcoxyle désignent quant à eux des groupements linéaires ou ramifiés comprenant de 1 à 9 atomes de carbone, de préférence de 1 à 6 atomes de carbone.

La présente invention a pour objet l'utilisation d'un composé de formule générale (I) 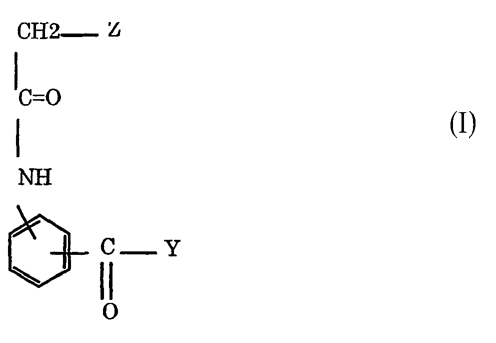 dans laquelle :
Z représente un atome d'halogène choisi dans le groupe constitué du chlore, du brome, de l'iode et du fluor,
Y est un substituant sélectionné dans le groupe constitué d'un groupe éthoxy, d'un groupe -NH-CO-NH-R dans lequel R est un hydrogène, un groupe alkyle ou un groupe aryle, et d'un groupe -N-R₁ dans lequel R₁ est un groupe alkyle ou un groupe hétérocyclique,
ou de ses sels d'addition pour la production d'un médicament en vue du traitement de maladies parasitaires, en particulier de protozooses, plus particulièrement de leishmanioses, du paludisme et des tripanosomiases.

La formule I ci-dessus recouvre trois types de composés, à savoir : les composés ortho de formule: 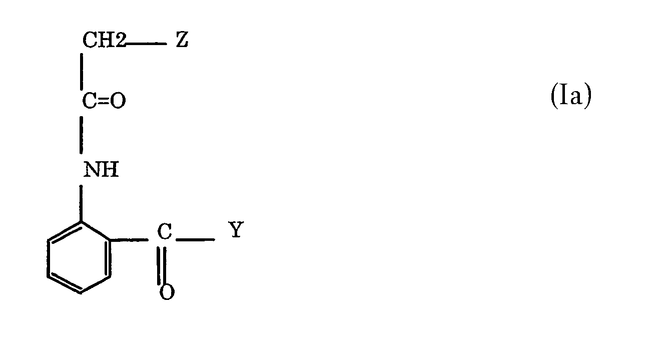 les composés méta de formule : 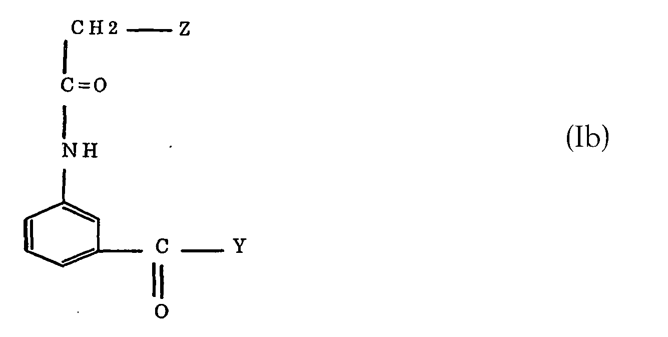 et les composés para de formule : 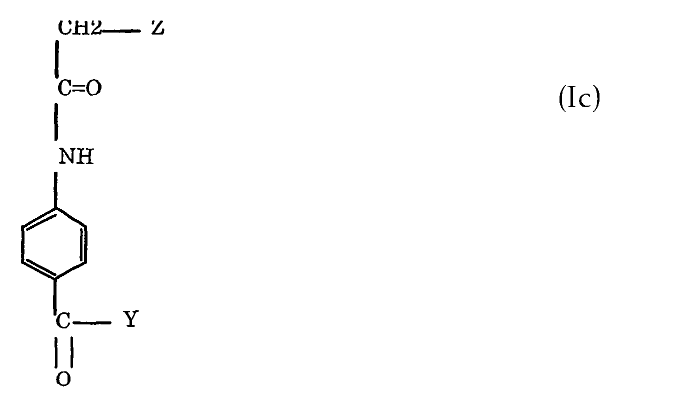 ainsi que leurs sels d'addition. Dans les formules Ia, Ib, Ic ci-dessus, Z et Y répondent aux mêmes définitions que celles fournies pour les composés de formule I.

Les composés de formule I ci-dessus peuvent ainsi être utilisés pour la production d'un agent anti-microtubulaire inhibant au moins partiellement la polymérisation de la tubuline du parasite sans inhiber celle de la cellule hôte.

La présente invention a encore pour objet l'utilisation d'un composé du type précité de formule générale (I), dans laquelle ce composé est associé à au moins un excipient ou véhicule inerte non toxique pharmaceutiquement acceptable permettant son administration par voie orale.

Ce composé peut se présenter sous forme d'une solution ou d'une suspension aqueuse ou à l'état sec de comprimés nus ou enrobés, de gélules, de capsules, de poudres.

L'invention a encore pour objet l'utilisation d'un composé de formule générale (I) du type précité, dans laquelle le composé est associé à au moins un excipient ou véhicule inerte non toxique pharmaceutiquement acceptable permettant son administration par voie topique sous forme d'application cutanée, transcutanée ou percutanée. Le composé peut ainsi se présenter sous forme d'une pommade, d'une crème, d'un gel dermique.

L'invention a encore pour objet l'utilisation d'un composé de formule générale (I) du type précité, dans laquelle le composé est associé à au moins un excipient ou véhicule inerte non toxique pharmaceutiquement acceptable permettant son administration par voie parentérale, nasale ou bronchique.

Généralement, la teneur en composé est choisie pour une administration s'échelonnant entre 1 mg et 5 g/24 heures, de préférence entre 1 mg et 500 mg/24 heures.

L'invention a encore pour objet un médicament comprenant un support physiologiquement acceptable et un composé de formule (I) du type ci-dessus ou un sel d'addition de celui-ci dans une quantité efficace pour traiter les maladies parasitaires, en particulier les protozooses, de préférence les leishmanioses, le paludisme et les tripanosomiases.

L'invention sera bien comprise à la lecture de la description suivante d'exemples de réalisation, en référence aux dessins annexés dans lesquels :
la figure 1 représente, sous forme de tableau, l'activité anti-Leishmania in vitro des molécules MF 29, MF 708, MF 56, MF 569, MF 191, GR 37, GR 38 et Glucantime (marque déposée), lesdites activités étant exprimées en µg/ml ;
la figure 2 représente les effets du MF 29 et du Glucantime (marque déposée) exprimés en pourcentage d'inhibition de la charge parasitaire par organe de source BALB/c infecté avec *L.Major ;*
les figures 3A à 3F représentent, sous forme de photographie, des altérations morphologiques induites par MF 29 (figures 3B, 3D, 3F) par comparaison à un groupe contrôle (figures 3A, 3C, 3E) ;
les figures 4A à 4D représentent, sous forme de courbe présentant en abscisse un temps exprimé en minutes et en ordonnée une absorbance à 345 nm, les effets d'inhibition de la polymérisation de tubuline de cerveau de boeuf par la vinblastine (figure 4A), le MF 191 (figure 4B) et le MF 29 à différents dosages (figure 4C et 4D) et
la figure 5 représente, sous forme de tableau, les pourcentages de mortalité obtenus sur des souris traités au MF 29 et infectées par Trypanosoma cruzi.

A titre d'exemple et d'illustration de l'invention, il est plus particulièrement présenté ci-après des résultats obtenus à partir des molécules de formules générales (II), (III), (IV), (V),

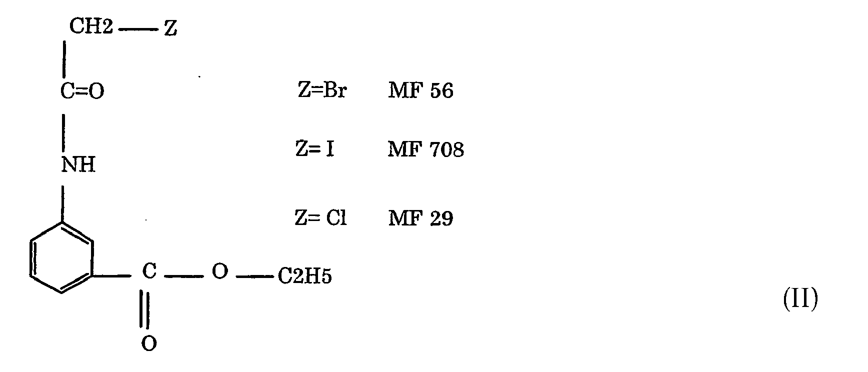

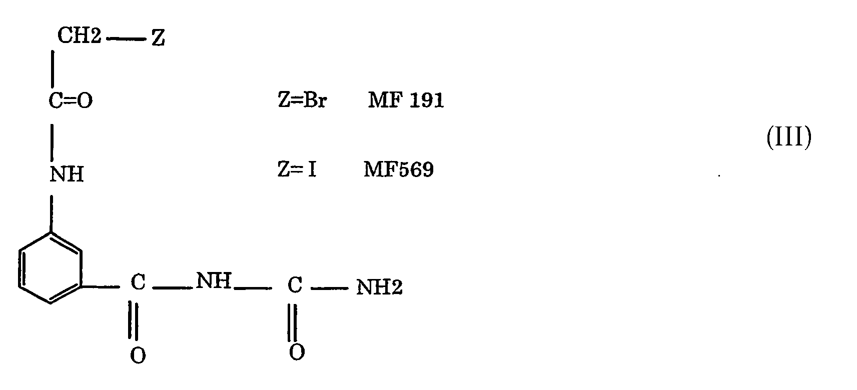

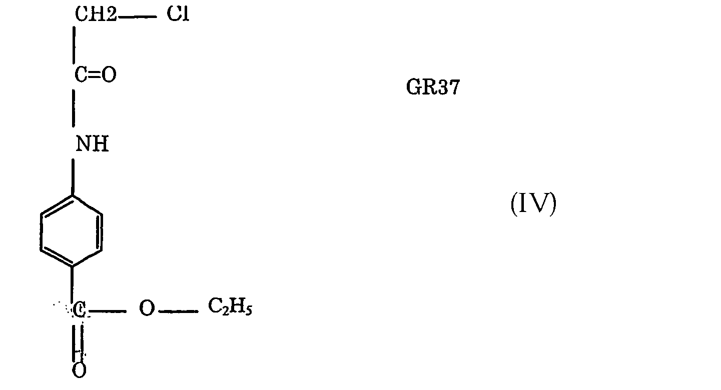

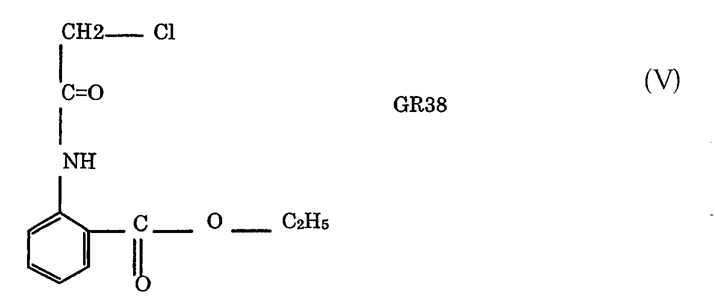

Ainsi:
- le composé MF 191 correspond à la 3-bromoacétamido benzoyl urée,
- le MF 569 correspond à la 3-iodoacétamido benzoyl urée,
- le MF 708 correspond au 3-iodoacétamido benzoate d'éthyle,
- le MF 56 correspond au 3-bromoacétamido benzoate d'éthyle,
- le MF 29 correspond au 3-chloroacétamido benzoate d'éthyle,
- le GR 37 correspond au 4-chloroacétamido benzoate d'éthyle et
- le GR 38 correspond au 2-chloroacétamido benzoate d'éthyle.

Il est décrit ci-après l'activité anti-Leishmania *in vitro* des molécules décrites ci-dessus.

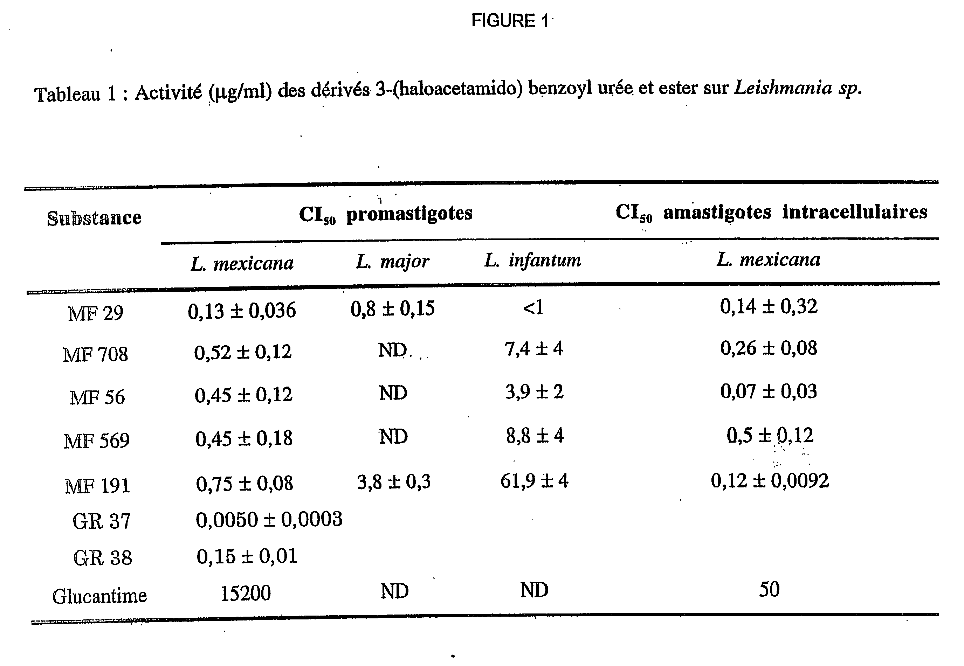

Les molécules ci-dessus ont été évaluées tout d'abord dans un test d'inhibition de prolifération du stade promastigote de *Leishmania* après 96 h de contact des promastigotes avec les molécules mises en solution dans un solvant : N,N diméthyl acétamide / propylène glycol / tween 80 (1:2:1). Un sel de tetrazolium (MTT) a été utilisé comme révélateur. Dans le cas de *L*. *mexicana,* les molécules ont été évaluées vis à vis du stade amastigote intracellulaire (macrophages de Balb/c), stade rencontré chez les patients. Le tableau 1 (figure 1) présente certaines des CI₅₀ des dérivés 3-haloacetamido benzoate urée, 3-haloacétamido benzoate d'éthyle, 4-haloacétamido benzoate d'éthyle et 2-chloroacétamido benzoate d'éthyle ainsi que, pour *L*. *mexicana,* celles de la molécule thérapeutique de référence, l'antimoniate de méglumine (GLUCANTIME) (marque déposée). Il est à noter que la CI₅₀ exprimée en µg de molécule active/ml de solvant correspond à la concentration de composé inhibant 50 % de la prolifération du parasite leishmania. Ces résultats montrent la forte activité du MF 29, similaire à celle du GR 38, qui est pour la forme amastigote 300 fois supérieure à celle du GLUCANTIME. Il doit toutefois être noté l'extraordinaire activité des composés de la forme para ou 4 haloacétamido benzoate d'éthyle comme l'illustre le GR 37 laissant entrevoir un grand potentiel dans le domaine de la thérapie des maladies parasitaires. Enfin, un essai de cytotoxicité réalisé sur lignée MRC5 (tissu normal de poumon de foetus) indique que la cytotoxicité du MF29 est 400 fois plus faible sur cette lignée que celle obtenue sur le parasite démontrant ainsi la faible toxicité du MF 29 sur les cellules humaines. De même, la cytotoxicité du GR37 est 1000 fois plus faible sur des macrophages murins que sur le parasite confirmant le fort potentiel de cette molécule.

L'activité anti-*Leishmania in vivo* de certaines molécules, en particulier de la molécule MF 29, a été également examinée.

Des promastigotes de *L*. *major* (2 x 10⁶) ou de *L. infantum* (10⁷) en phase stationnaire sont inoculés respectivement par voie sous cutanée dans la patte arrière droite ou par voie intraveineuse dans la veine caudale. Les souris sont traitées par voie intra-péritonéale une fois par jour à raison de 10 mg/kg de MF 29 en solution dans un solvant : N,N diméthyl acétamide / propylène glycol / tween 80 (1:2:1) durant 10 jours. L'antimoniate de meglumine (Glucantime-Specia (marque déposée) Rhone-Poulenc, Paris, France) contenant 85 mg d'antimoine pentavalent par ml est administré par voie sous cutanée selon le même schéma thérapeutique. Le groupe contrôle reçoit le solvant du MF 29 et de la solution saline à 0,9 % par voie intrapéritonéale.

Pour le modèle cutané, l'efficacité du traitement est évaluée par mesure de l'oedème au niveau de la patte infectée à l'aide d'un pied à coulisse électronique (réf. 56371 ; Polylabo ; Strasbourg, France), et rapportée par rapport à l'épaisseur de la patte contre-latérale. Les souris sont sacrifiées neuf semaines post-infection. Un fragment de derme, de la rate, du foie et du ganglion poplité drainant la lésion sont conservés pour déterminer la charge parasitaire *in situ*. En ce qui concerne le modèle de leishmaniose viscérale, le même protocole est réalisé sur la rate et le foie. La charge parasitaire est exprimée par le log₁₀ du nombre de parasites par gramme du tissu.

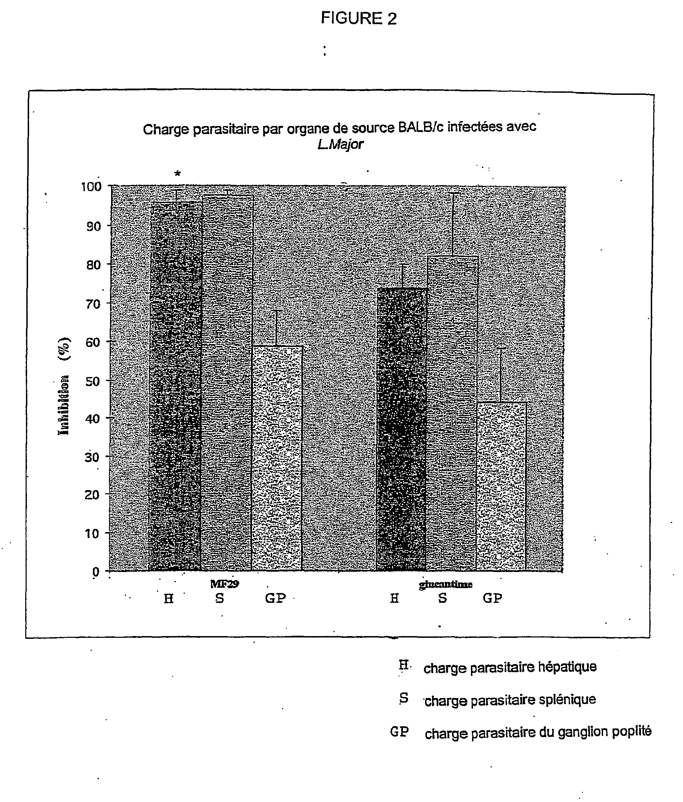

Ces essais montrent une forte activité du composé MF 29 sur l'infection à *L*. *major* chez la souris Balb/c. Le traitement entraîne une réduction de la charge parasitaire hépatique de 96 %, de la charge splénique de 97 % et de celle du ganglion poplité de 59 %. Dans ce même modèle, la molécule de référence, le glucantime, entraîne une réduction plus faible : les valeurs s'élèvent respectivement à 74,82 et 44 % (figure 2). Dans le modèle viscéral, le glucantime et le MF 29 font preuve d'une efficacité similaire. La réduction de la charge parasitaire au niveau du foie est de 25 et 30 % tandis qu'au niveau de la rate, cette réduction s'élève respectivement à 56 et 66 % pour le MF 29 et le Glucantime.

Les études in vivo, en prenant pour modèle la souris infectée, confirment à la fois l'effet cytotoxique important sur la prolifération du parasite et la faible toxicité du produit pour la souris.

L'étude des altérations morphologiques induites par le MF 29 ont également été étudiées.

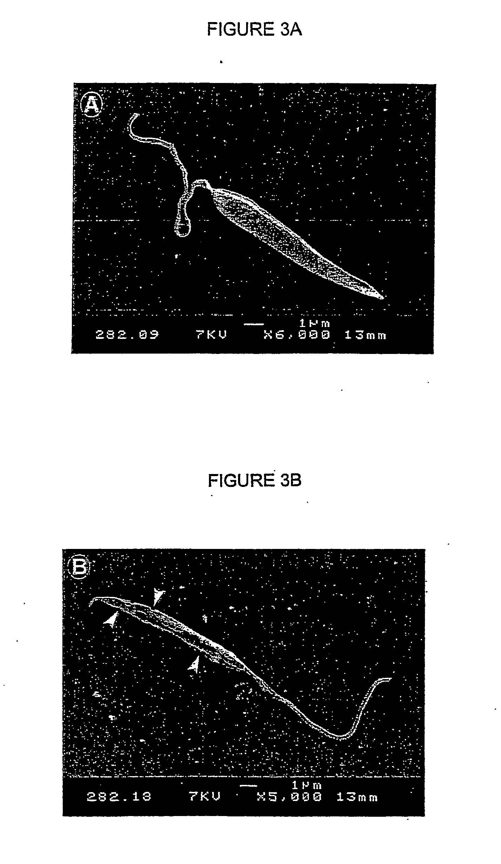

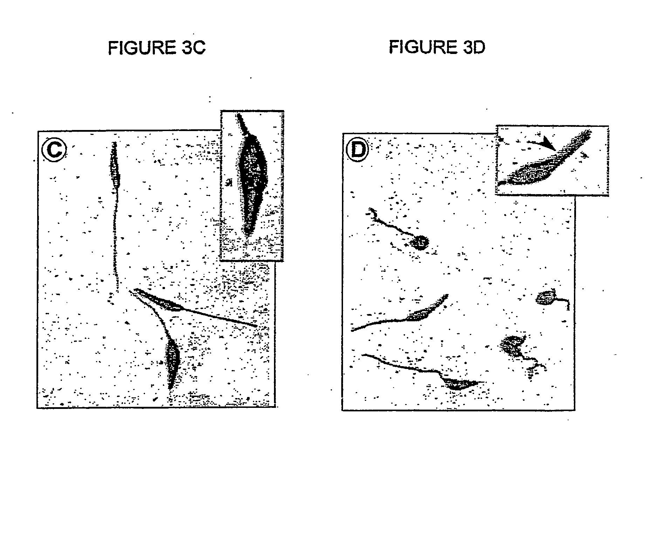

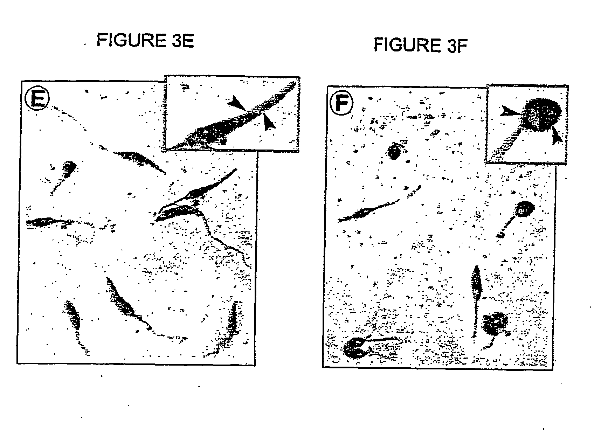

Après traitement d'une heure par le MF29 à 5 µg/ml et observation en microscopie électronique à balayage (JEOL, ISM 6400F), les promastigotes de *L*. *mexicana* présentent de multiples pores (voir flèches) au niveau du corps cellulaire tandis que, contrairement à d'autres drogues anti-tubuline, le flagelle ne semble pas perturbé [figure 3, contrôle (A) et traité (B)].

Enfin, l'action anti-microtubulaire du MF 29 a également été étudiée. Il a d'abord été étudié dans un premier temps l'altération des réseaux de microtubules de promastigote de *Leishmania* après traitement au MF 29.

Des anticorps dirigés contre la tubuline α et une protéine reliée immunologiquement à la protéine neuronale MAP₂, ont montré que (figure 3), sous l'effet de la drogue, certaines classes de microtubules étaient désorganisées. La couronne de microtubules sous-corticaux était discontinue [figure 3, contrôle (C) et traitée (D2)] et certains microtubules par la protéine MAP₂ like étaient partiellement dépolymérisés [figure 3, contrôle (E) et traitée (F)]
La technique utilisée a consisté à fixer les parasites, puis à les incuber en présence d'un premier anticorps (anti-tubuline α ou anti-MAP₂) toute la nuit, puis le lendemain, après lavage, à incuber en présence d'un deuxième anticorps dirigé contre le premier, couplé à la biotine. Un système amplificateur streptavidine-peroxydase est utilisé, puis le signal est détecté par l'aminoéthylcarbazole. La visualisation des cellules parasitaires est ensuite réalisée grâce à un microscope relié à une caméra tri-CDD (Lhesa electronic system, Rungis, France).

L'action anti-microtubulaire du MF 29 est également comparée à celle du MF 191 et à celle de la vinblastine sur l'ensemble des tubulines de cerveau de boeuf.

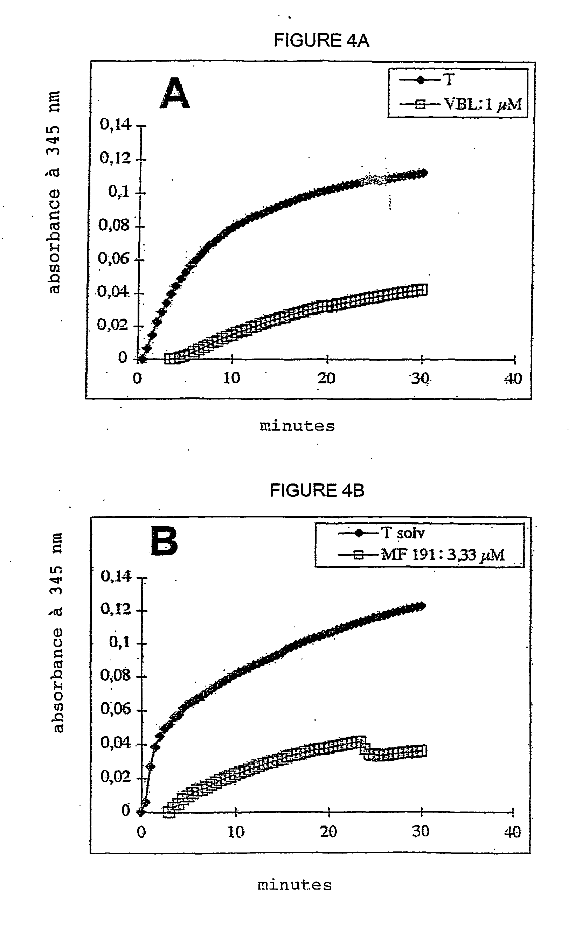

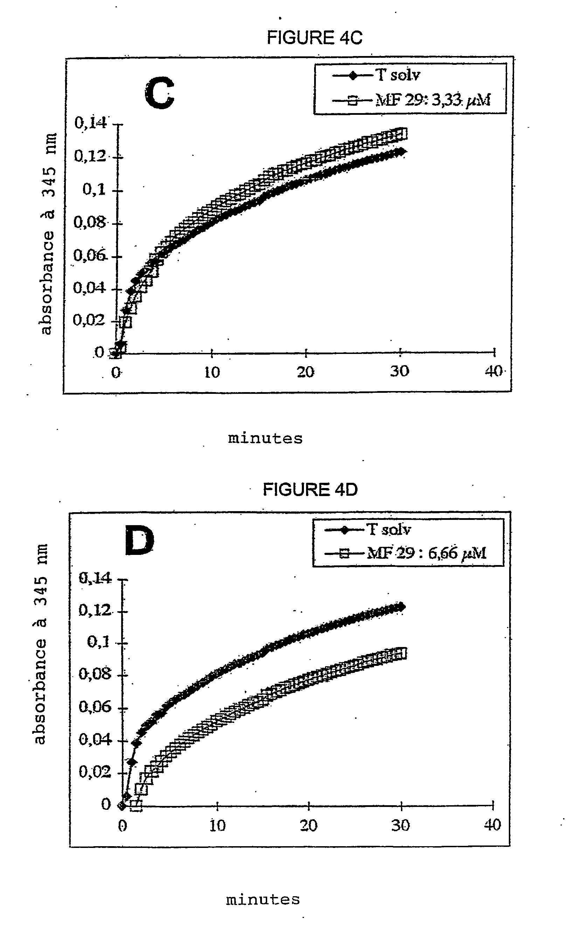

Les expériences effectuées pour mesurer la capacité de polymérisation *in vitro* de la tubuline sont réalisées à l'aide d'un spectrophotomètre équipé d'un système de bain circulant à 37°C et d'un chargeur de cuve automatique permettant la mesure simultanée de plusieurs essais. La densité optique qui augmente avec la polymérisation de l'ensemble des tubulines de cerveau selon Lebanski et al, 1973, que l'on induit avec le promoteur MAP₂ est mesurée à 345 nm toutes les trente secondes. La figure 4 montre que le MF 29 est un faible inhibiteur de la polymérisation de tubuline de cerveau du boeuf (Figure 4C). Pour avoir une inhibition significative (Figure 4D), il faut doubler la concentration de drogue. Même dans ce cas, cette inhibition reste inférieure à celle produite par le MF 191 (figure 4B) ou par la vinblastine (Figure 4A). Le MF 29 présente donc une action sélective d'inhibition de la polymérisation de tubuline en agissant principalement sur la tubuline du parasite et non sur celle de la cellule hôte.

Le MF29 présente donc l'avantage de ne pas altérer les microtubules de mammifères. A l'inverse d'autres antimicrotubulaires à activité antiparasitaire sont associés à une forte toxicité sur des macrophages humains (tubulozoles) ou sur différentes lignées de cellules mammifères (taxoïdes, vinca alcaloïdes). Par ailleurs, les faibles niveaux d'activité (CI50 élevées) de ces composés sur les formes amastigotes de *Leishmania* ou sur la tubuline parasitaire indiquent l'intérêt majeur des composés ci-dessus décrits en exemple.

Les études approfondies montrent que le mécanisme d'action du MF 29 implique une différence d'activité selon la classe de microtubules. Alors que la relation entre l'hétérogénéité des microtubules et l'action des drogues antimicrotubulaires, tels que les tubulozoles, n'a jamais été abordée, le MF 29 a un effet plus marqué quand des protéines associées aux tubulines (MAP) sont liées aux microtubules. A ce titre, il a été identifié pour la première fois chez *Leishmania* des protéines immunologiquement apparentées à MAP2. Connaissant le rôle important des MAP2 dans les processus de division et de différenciation cellulaire, le MF29 pourrait agir très efficacement contre ces processus en altérant les microtubules MAP2-like du parasite.

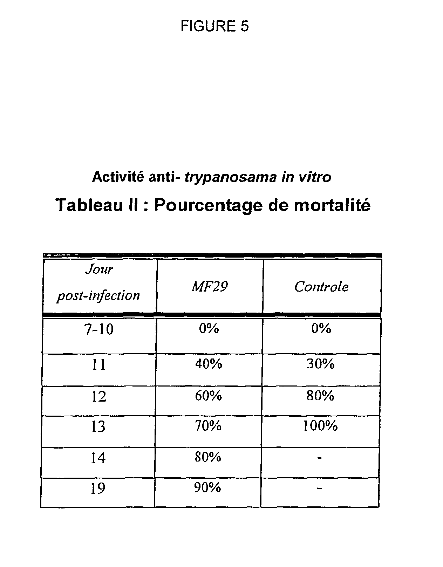

L'activité anti-*Trypanosoma in vivo* du MF 29 a également été étudiée. Des souris sont infectées par *Trypanosoma cruzi* souche péruvienne par voie intraveineuse. Quatre jours post-infection, les souris sont traitées par le MF 29 par voie intra-peritonéale une fois par jour à raison de 10 mg /Kg durant 10 jours. Le groupe contrôle reçoit le solvant. L'efficacité du traitement est évaluée par mesure de la parasitémie et suivi de la mortalité. Ces essais montrent une réduction maximale de la parasitémie au 8ème jour. En effet, la charge est de 200 trypomastigotes par 50 champs pour le groupe traité par le MF 29 contre 680 dans le groupe contrôle, soit une réduction de la charge parasitaire de 70 %. Au 10 ème jour, la parasitémie est équivalente pour les deux groupes. Le traitement entraîne par ailleurs un retard dans la courbe de mortalité comme l'illustre la figure 5. Les résultats préliminaires d'une utilisation du MF 29 à 20 mg/kg montrent une réduction plus importante de la mortalité. Eu égard aux faibles doses utilisées, des résultats satisfaisant pourraient être obtenus dans le cas de souches moins virulentes ou pour les souches ci-dessus à des doses plus élevées.

L'activité anti- *Plasmodium in vitro* du MF 29 a également été étudiée. Le MF 29 a été évalué dans un essai d'inhibition de la prolifération de *Plasmodium falciparum* (espèce mortelle) dans un modèle intra-érythrocytaire par mesure de l'incorporation d'hypoxanthine tritiée. Le MF 29 montre une forte activité avec une CI50 de 0.1 µg/ml sur une souche chloroquino-sensible (FCB1-Columbia) mais également sur une souche résistante (souche Nigerian) à la chloroquine.

Quelques exemples de compositions pharmaceutiques sont encore fournis ci-après :

### Exemple A :

Comprimé pour le traitement des parasitoses
Composés dosés à 10 mg de molécule active
Formule de préparation pour 1000 comprimés

| | |
|---|---|
| Molécule active | 10 g |
| Amidon de blé | 35 g |
| Amidon de mais | 65 g |
| Lactose | 65 g |
| Stéarate de magnésium | 2 g |
| Silice | 1 g |
| Hydroxypropylcellulose | 2 g |

### Exemple B :

Pommade destinée au traitement des parasitoses
Formule de préparation pour 100 kg

| | |
|---|---|
| Molécule active | 1000 g |

Excipient en quantité suffisante pour 100 kg

L'excipient peut être choisi dans le groupe ci-dessous : Alcools cétylique, stéarylique, isopropylique ; lanoline, monostéréate de polyéthylène glycol, eau distillée de laurier cerise.

Il est bien évident que les exemples ci-dessus sont fournis uniquement à titre d'illustration de l'invention.

La présente invention a également pour objet l'utilisation de composés de formule générale (I) pour la préparation d'un médicament pour un procédé d'inhibition de la polymérisation de la tubuline par mise en contact d'un système tel qu'un parasite contenant de la tubuline et induisant une maladie parasitaire avec une quantité efficace d'un composé de formule générale I ayant la structure ci-dessus.

La présente invention a également pour objet l'utilisation de composés de formule générale (I) pour la préparation d'un médicament pour un procédé d'inhibition de la prolifération des parasites induisant les leishmanioses, le paludisme et les tripanosomiases, comprenant l'administration d'une quantité thérapeutiquement active des composés anti-microtubulaires de formule générale I décrits ci-dessus.

La présente invention a également pour objet l'utilisation de composés de formule générale (I) pour la préparation d'un médicament pour un procédé d'inhibition in vitro ou in vivo de la croissance et/ou de la prolifération de parasites induisant des maladies parasitaires telles que les leishmanioses, le paludisme et les tripanosomiases, ce procédé comprenant la mise en contact des parasites avec une quantité efficace d'un agent anti-microtubulaire de formule générale I apte à inhiber la polymérisation de la tubuline tel que décrit ci-dessus. Le parasite peut être logé chez un patient.

## Revendications

1. Utilisation d'un composé de formule générale (I) 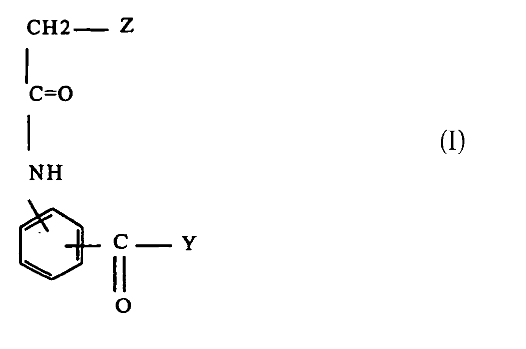 dans laquelle
Z représente un atome d'halogène choisi dans le groupe constitué du chlore, du brome, de l'iode et du fluor,
Y est un substituant sélectionné dans le groupe constitué d'un groupe éthoxy, d'un groupe -NH-CO-NH-R dans lequel R est un hydrogène, un groupe alkyle ou un groupe aryle, et d'un groupe -N-R₁ dans lequel R₁ est un groupe alkyle ou un groupe hétérocyclique,
ou de ses sels d'addition pour la production d'un médicament en vue du traitement de maladies parasitaires, en particulier de protozooses, plus particulièrement des leishmanioses, du paludisme et des tripanosomiases.

2. Utilisation d'un composé de formule générale (I) 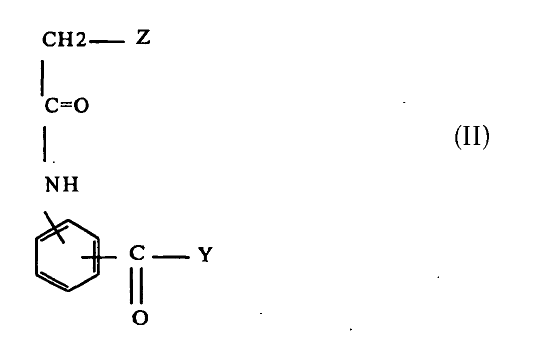 dans laquelle
Z représente un atome d'halogène choisi dans le groupe constitué du chlore, du brome, de l'iode et du fluor,
Y est un substituant sélectionné dans le groupe constitué d'un groupe éthoxy, d'un groupe -NH-CO-NH-R dans lequel R est un hydrogène, un groupe alkyle ou un groupe aryle, et d'un groupe -N-R₁ dans lequel R₁ est un groupe alkyle ou un groupe hétérocyclique,
ou de ses sels d'addition pour la production d'un médicament incluant au moins un agent anti-microtubulaire actif pour le traitement de protozooses du système réticulo endothélial des mammifères.

3. Utilisation d'un composé de formule générale (I) 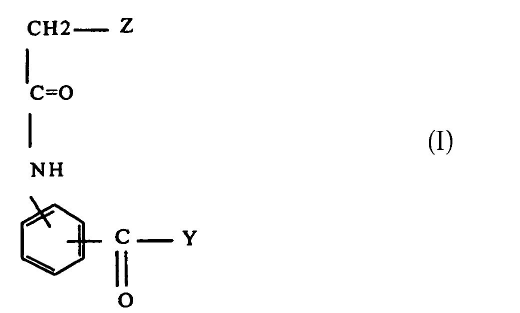 dans laquelle
Z représente un atome d'halogène choisi dans le groupe constitué du chlore, du brome, de l'iode et du fluor,
Y est un substituant sélectionné dans le groupe constitué d'un groupe éthoxy, d'un groupe -NH-CO-NH-R dans lequel R est un hydrogène, un groupe alkyle ou un groupe aryle, et d'un groupe -N-R₁ dans lequel R₁ est un groupe alkyle ou un groupe hétérocyclique,
ou de ses sels d'addition pour la préparation d'un médicament pour le traitement des maladies parasitaires en inhibant au moins partiellement la polymérisation de la tubuline du parasite sans inhiber celle de la cellule hôte.

4. Utilisation d'un composé selon l'une des revendications 1 à 3,
dans laquelle le composé est associé à au moins un excipient ou véhicule inerte, non toxique, pharmaceutiquement acceptable permettant son administration par voie orale.

5. Utilisation d'un composé selon la revendication 4,
dans laquelle le composé se présente sous forme d'une solution ou d'une suspension aqueuse ou à l'état sec de comprimés nus ou enrobés, de gélules, de capsules, de poudres.

6. Utilisation d'un composé selon l'une des revendications 1 à 3,
dans laquelle le composé est associé à au moins un excipient ou véhicule inerte non toxique pharmaceutiquement acceptable permettant son administration par voie topique sous forme d'application cutanée, transcutanée ou percutanée.

7. Utilisation d'un composé selon la revendication 6,
dans laquelle le composé est sous forme d'une pommade, d'une crème ou d'un gel dermique.

8. Utilisation d'un composé selon l'une des revendications 1 à 3,
dans laquelle le composé est associé au moins un excipient ou véhicule inerte non toxique pharmaceutiquement acceptable permettant son administration par voie parentérale, nasale ou bronchique.

9. Utilisation d'un composé selon l'une des revendications 1 à 8,
dans laquelle la teneur en composé est choisie pour une administration s'échelonnant entre 1 mg et 5 g/24 heures, de préférence entre 1 mg et 500 mg/24 heures.

## Claims

1. Use of a compound of the general formula (I) 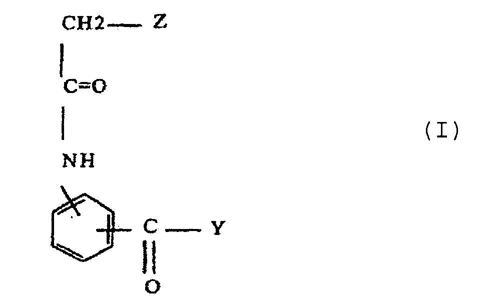 in which
Z represents a halogen atom selected from the group composed of chlorine, bromine, iodine and fluorine,
Y is a substituent selected from the group composed of an ethoxy group, a group -NH-CO-NH-R in which R is hydrogen, an alkyl group or an aryl group, and a group -N-R₁ in which R₁ is an alkyl group or a heterocyclic group,
or its addition salts, in the production of a medicament for the treatment of parasitic diseases, in particular protozooses, more particularly leishmanioses, paludism and trypanosomiases.

2. Use of a compound of the general formula (I) 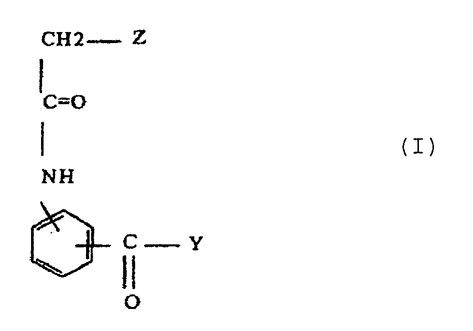 in which
Z represents a halogen atom selected from the group composed of chlorine, bromine, iodine and fluorine,
Y is a substituent selected from the group composed of an ethoxy group, a group -NH-CO-NH-R in which R is hydrogen, an alkyl group or an aryl group, and a group -N-R₁ in which R₁ is an alkyl group or a heterocyclic group,
or its addition salts, in the production of a medicament that includes at least one anti-microtubular agent active for the treatment of protozooses of the reticuloendothelial system of mammals.

3. Use of a compound of the general formula (I) 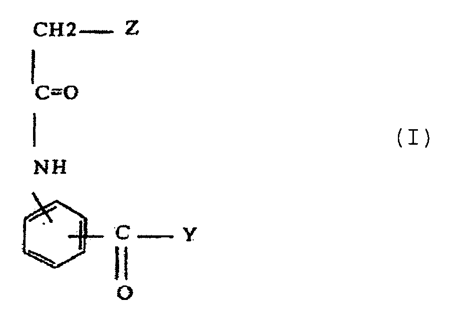 in which
Z represents a halogen atom selected from the group composed of chlorine, bromine, iodine and fluorine,
Y is a substituent selected from the group composed of an ethoxy group, a group -NH-CO-NH-R in which R is hydrogen, an alkyl group or an aryl group, and a group -N-R₁ in which R₁ is an alkyl group or a heterocyclic group,
or its addition salts, in the preparation of a medicament for the treatment of parasitic diseases by inhibiting at least partially the polymerisation of the tubulin of the parasite without inhibiting that of the host cell.

4. Use of a compound according to any one of claims 1 to 3,
wherein the compound is in association with at least one pharmaceutically acceptable, inert, non-toxic excipient or carrier permitting its administration by the oral route.

5. Use of a compound according to claim 4,
wherein the compound is in the form of a solution or of an aqueous suspension or is in the dry state of uncoated or coated tablets, of gelatin capsules, of powders.

6. Use of a compound according to any one of claims 1 to 3,
wherein the compound is in association with at least one pharmaceutically acceptable, inert, non-toxic excipient or carrier permitting its administration by the topical route in the form of cutaneous, transcutaneous or percutaneous administration.

7. Use of a compound according to claim 6,
wherein the compound is in the form of an ointment, a cream or a dermal gel.

8. Use of a compound according to any one of claims 1 to 3,
wherein the compound is in association with at least one pharmaceutically acceptable, inert, non-toxic excipient or carrier permitting its administration by the parenteral, nasal or bronchial route.

9. Use of a compound according to any one of claims 1 to 8,
wherein the content of compound is chosen for an administration ranging from 1 mg to 5 g/24 hours, preferably from 1 mg to 500 mg/24 hours.

## Patentansprüche

1. Verwendung einer Verbindung der allgemeinen Formel (I) 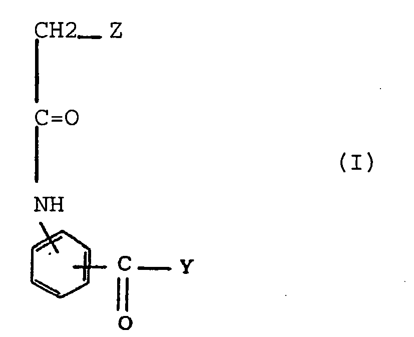 worin Z ein aus der Gruppe bestehend aus Chlor, Brom, Iod und Fluor ausgewähltes Halogenatom bedeutet, Y ein aus der Gruppe bestehend aus einer Ethoxygruppe, einer -NH-CO-NH-R-Gruppe, worin R ein Wasserstoff ist, einer Alkylgruppe oder einer Arylgruppe und einer -N-R₁-Gruppe, worin R₁ eine Alkylgruppe oder eine heterozyklische Gruppe ist, ausgewählter Substituent ist, oder ihrer Additionssalze für die Herstellung eines Medikamentes zur Behandlung parasitärer Krankheiten, insbesondere von Protozoonosen und davon insbesondere von Leishmaniose, Malaria und Schlafkrankheit.

2. Verwendung einer Verbindung der allgemeinen Formel (I) 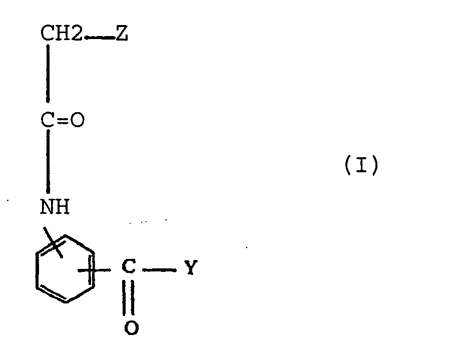 worin Z ein aus der Gruppe bestehend aus Chlor, Brom, Iod und Fluor ausgewähltes Halogenatom bedeutet, Y ein aus der Gruppe bestehend aus einer Ethoxygruppe, einer -NH-CO-NH-R-Gruppe, worin R ein Wasserstoff ist, einer Alkylgruppe oder einer Arylgruppe und einer -N-R₁-Gruppe, worin R₁ eine Alkylgruppe oder eine heterozyklische Gruppe ist, ausgewählter Substituent ist, oder ihrer Additionssalze für die Herstellung eines Medikamentes mit mindestens einem antimikrotubulären Agens zur Behandlung von Protozoonosen des retikuloendothelialen Systems von Säugern.

3. Verwendung einer Verbindung der allgemeinen Formel (I) 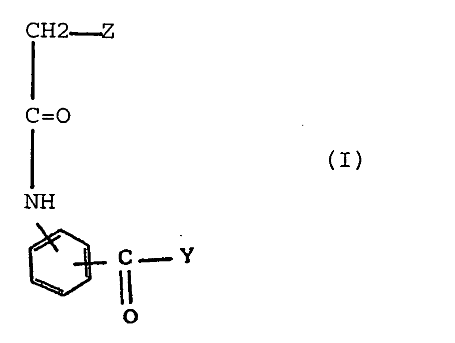 worin Z ein aus der Gruppe bestehend aus Chlor, Brom, Iod und Fluor ausgewähltes Halogenatom bedeutet, Y ein aus der Gruppe bestehend aus einer Ethoxygruppe, einer -NH-CO-NH-R-Gruppe, worin R ein Wasserstoff ist, einer Alkylgruppe oder einer Arylgruppe und einer -N-R₁-Gruppe, worin R₁ eine Alkylgruppe oder eine heterozyklische Gruppe ist, ausgewählter Substituent ist, oder ihrer Additionssalze für die Zubereitung eines Medikamentes zur Behandlung parasitärer Krankheiten durch zumindest teilweiser Hemmung der Polymerisation des Tubulins des Parasiten ohne Hemmung der Polymerisation des Tubulins der Wirtszelle.

4. Verwendung einer Verbindung nach einem der Ansprüche 1 bis 3, worin die Verbindung mindestens einem inerten, ungiftigen und pharmazeutisch annehmbaren Bindemittel oder Trägerstoff zugeordnet ist, der ihre orale Verabreichung ermöglicht.

5. Verwendung einer Verbindung nach Anspruch 4, worin die Verbindung als Lösung oder wässrige Suspension oder im trockenen Zustand als nicht-überzogene oder überzogene Tablette, als Gelatinekapsel, als Hartkapsel oder als Pulver vorliegt.

6. Verwendung einer Verbindung nach einem der Ansprüche 1 bis 3, worin die Verbindung mindestens einem reaktionsträgen, ungiftigen und pharmazeutisch annehmbaren Bindemittel oder Trägerstoff zugeordnet ist, der ihre lokale Verabreichung durch kutane, transkutane oder perkutane Anwendung ermöglicht.

7. Verwendung einer Verbindung nach Anspruch 6, worin die Verbindung als Salbe, Creme oder Hautgel vorliegt.

8. Verwendung einer Verbindung nach einem der Ansprüche 1 bis 3, worin die Verbindung mindestens einem reaktionsträgen, ungiftigen und pharmazeutisch annehmbaren Bindemittel oder Trägerstoff zugeordnet ist, der ihre parenterale, nasale oder bronchiale Verabreichung ermöglicht.

9. Verwendung einer Verbindung nach einem der Ansprüche 1 bis 8, worin der Verbindungsgehalt für eine Verabreichung zwischen 1 mg und 5 g/24 Stunden und vorzugsweise zwischen 1 mg und 500 mg/24 Stunden gewählt wird.
